(19) Europäisches Patentamt
European Patent Office
Office européen des brevets

(11) EP 1 631 267 B1

(12) **EUROPEAN PATENT SPECIFICATION**

(45) Date of publication and mention of the grant of the patent:
**02.01.2008 Bulletin 2008/01**

(21) Application number: **04776158.0**

(22) Date of filing: **28.05.2004**

(51) Int Cl.:
***A61K 31/33*** (2006.01) ***A61K 31/505*** (2006.01)

(86) International application number:
**PCT/US2004/016874**

(87) International publication number:
**WO 2005/002506 (13.01.2005 Gazette 2005/02)**

(54) **METHOD FOR TREATING DISEASES USING HSP90-INHIBITING AGENTS IN COMBINATION WITH ANTIMETABOLITES**

VERFAHREN ZUR BEHANDLUNG VON KRANKHEITEN MIT HSP90-HEMMENDEN MITTELN IN KOMBINATION MIT ANTIMETABOLITEN

METHODE DE TRAITEMENT DE MALADIES METTANT EN OEUVRE DES AGENTS INHIBANT HSP90 CONJOINTEMENT AVEC DES ANTIMETABOLITES

(84) Designated Contracting States:
**AT BE BG CH CY CZ DE DK EE ES FI FR GB GR HU IE IT LI LU MC NL PL PT RO SE SI SK TR**

(30) Priority: **30.05.2003 US 474906 P**
**27.05.2004 US 856342**

(43) Date of publication of application:
**08.03.2006 Bulletin 2006/10**

(73) Proprietor: **Kosan Biosciences, Inc.**
**Hayward, CA 94545 (US)**

(72) Inventors:
- **JOHNSON, Robert, Jr.**
**Lafayette, California 94549 (US)**
- **ZHOU, Yiqing**
**Lafayette, California 94549 (US)**
- **MÜLLER, Thomas**
**San Francisco, California 94114 (US)**

(74) Representative: **Roques, Sarah Elizabeth**
**J.A. Kemp & Co.**
**14 South Square**
**Gray's Inn**
**London WC1R 5JJ (GB)**

(56) References cited:
**WO-A-00/37050** **WO-A-02/36574**
**WO-A-03/013430** **US-A1- 2004 053 909**

- **DATABASE BIOSIS [Online] BIOSCIENCES INFORMATION SERVICE, PHILADELPHIA, PA, US; July 2003 (2003-07), ZHOU YIQING ET AL: "Synergistic cytotoxicity of 17-allylamino-17-demethoxygeldanamycin (17-AAG) and 5-fluorouracil in human colon cancer cell lines." XP002379273 Database accession no. PREV200300441941 & PROCEEDINGS OF THE AMERICAN ASSOCIATION FOR CANCER RESEARCH ANNUAL MEETING, vol. 44, July 2003 (2003-07), page 153, 94TH ANNUAL MEETING OF THE AMERICAN ASSOCIATION FOR CANCER RESEARCH; WASHINGTON, DC, USA; JULY 11-14, 2003 ISSN: 0197-016X**
- **LIAO Z ET AL: "SYNERGISTIC EFFECTS OF GELDANAMYCIN AND ANTITUMOR DRUGS" YAO HSUEH HSUEH PAO - ACTA PHARMACEUTICA SINICA, BEIJING, CN, vol. 36, no. 8, 2001, pages 569-575, XP008045475 ISSN: 0513-4870**
- **DATABASE MEDLINE [Online] KELLAND L. ET AL: 'DT-Diaphorase expression and tumor cell sensitivity to 17-allylamino, 17-demethoxygeldanamycin, an inhibitor of heat shock protein 90', XP002997356 Retrieved from NLM Database accession no. (NLM10564678) & J NATL CANCER INSTITUTE vol. 91, no. 22, 17 November 1999, pages 1940 - 1949**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 631 267 B1

- **DATABASE DIALOG (CANCERLIT) [Online] SOGA S ET AL: 'KF25706, a Novel Oxime Derivative of Radicicol, Exhibits in Vivo Antitumor Activity via Selective Depletion of Hsp90 Binding Signaling Molecules', XP002997357 Retrieved from NLM Database accession no. (99310530) & CANC RES vol. 59, no. 12, 15 June 1999, pages 2931 - 2938**
- **DATABASE MEDLINE [Online] WEIN A. ET AL: 'Weekly 24-h infusion of high-dose 5-fluorouracil (5-FU) with folinic acid (FA) in adjuvant therapy of colon cancer', XP002997358 Retrieved from NLM Database accession no. (NLM11253506) & ZEITSCHRIFT FUR GASTROENTEROLOGIE vol. 39, no. 2, February 2001, pages 153 - 156**

**Description**

BACKGROUND OF THE INVENTION

*Field of the Invention*

This invention relates to methods for treating cancer in which an inhibitor of Heat Shock Protein 90 ("HSP90") is combined with an antimetabolite. More particularly, this invention relates to combinations of the HSP90 inhibitor geldanamycin and its derivatives, especially 17-alkylamino-17-desmethoxygeldanamycin ("17-AAG") and 17-(2-dimethylaminoethyl)amino-17-desmethoxygeldanamycin ("17-DMAG"), with an antimetabolite (*e.g.,* 5-fluorouracil and gemcitabine).

The antimetabolite used in accordance with the invention is gemcitabine.

*References*

Agnew et al., "Clinical pharmacokinetics of 17-(allylamino)-17-demethoxygeldanamycin and the active metabolite 17-(amino)-17-demethoxygctdanamycin given as a one-hour infusion daily for 5 days." AACR, 2002.

An et al., "Depletion of p185erbB2, Raf-1 and mutant p53 proteins by geldanamycin derivatives correlates with antiproliferative activity." Cancer Chemother. Pharmacol. 40:60-64,1997.

Bagatell et al., "Induction of a heat shock factor 1-dependent stress response alters the cytotoxic activity of hsp90-binding agents." Clin. Cancer Res. 6:3312-3318, 2000.

Bagatell et al., "Destabilization of steroid receptors by heat shock protein 90-binding drugs: a ligand-independent approach to hormonal therapy of breast cancer." Clin. Cancer Res. 7:2076-2084, 2001.

Banerji et al., "A pharmacokinetically (PK)-pharmacodynamically (PD) driven Phase I trial of the HSP90 molecular chaperone inhibitor 17-allylamino-17-demethoxygeldanamycin (17-AAG)." AACR, 2002.

Barent et al., "Analysis of FKBP51/FKBP52 chimeras and mutants for Hsp90 binding and association with progesterone receptor complexes." Mol. Endocrinol. 12:342-354, 1998.

Bilodeau et al., "Tyrosine kinase inhibitors." U.S. Patent No. 6,245,759 issued June 12, 2001.

Citri et al., "Drug-induced ubiquitylation and degradation of ErbB receptor tyrosine dinases: implications for cancer chemotherapy." EMBO Journal 21:2407-2417, 2002.

Egorin et al., "Metabolism of 17-(allylamino)-17-demethoxygeldanamycin (NSC 330507) by murine and human hepatic preparations." Cancer Res. 58:2385-2396, 1998.

Fraley et al., "Tyrosine kinase inhibitors." U.S. Patent No. 6,306,874 issued October 23, 2001.

Fraley et al., "Tyrosine kinase inhibitors." U.S. Patent No. 6,313,138 issued November 6, 2001.

Gaidigk et al., "NAD(P)H:quinone oxidoreductase: polymorphisms and allele frequencies n Caucasian, Chinese and Canadian Native Indian and Inuit populations." Pharmacogenetics 8:305-313, 1998.

Gelmon et al., "Anticancer agents targeting signaling molecules and cancer cell environment: challenges for drug development?" J. Natl. Cancer Inst. 91:128 1-1287,1999.

Goetz et al., "The Hsp90 chaperone complex as a novel target for cancer therapy." Ann. Oncol. 14:1169-1176, 2003.

Goh et al., "Explaining interindividual variability of docetaxel pharmacokinetics and pharmacodynamics in Asians through phenotyping and genotyping strategies." J. Clin. Oncol. 20:3683-3690, 2002.

Grenert et al., "The amino-terminal domain of heat shock protein 90 (hsp90) that binds geldanamycin is an ATP/ADP switch domain that regulates hsp90 conformation." J. Biol. Chem. 272:23843-23850,1997.

Johnson and Toft, "Binding of p23 and hsp90 during assembly with the progesterone receptor." Mol. Endocrinol. 9:670-678, 1995.

Hartl and Hayer-Hartl, "Molecular chaperones in the cytosol: from nascent chain to folded protein." Science 195:1852-1858, 2002.

Hegde et al., "Short circuiting stress protein expression via a tyrosine kinase inhibitor, herbimycin A." J. Cell Physiol. 165:186-200,1995.

Hustert et al., "The genetic determinants of the CYP3A5 polymorphism." Pharmacogenetics 11:773-779, 2001.

Kelland et al., "DT-Diaphorase expression and tumor cell sensitivity to 17-allylamino, 17-demethoxygeldanamycin, an inhibitor of heat shock protein 90." J. Natl. Cancer Inst. 91:1940-1949,1999.

Kuehl et al., "Sequence diversity in CYP3A promoters and characterization of the genetic basis of polymorphic CYP3A5 expression." Nat. Genet. 27:383-391, 2001.

Lawson et al., "Geldanamycin, an hsp90/GRP94-binding drug, induces increased transcription of endoplasmic reticulum (ER) chaperones via the ER stress pathway." J. Cell Physiol. 174:170-178, 1998.

Lin et al., "Co-regulation of CYP3A4 and CYP3A5 and contribution to hepatic and intestinal midazolam metabolism." Mol. Pharmacol. 62:162-172, 2002.

Morimoto et al., "The heat-shock response: regulation and function of heat-shock proteins and molecular chaperones." Essays Biochem 32:17-29, 1997.

Munster et al., "Phase I trial of 17-(allylamino)-17-demethoxygeldanamycin (17-AAG) in patients with advanced solid malignancies." Proc. Am. Soc. Clin. Oncol, 83a, 2001.

Munster et al., "Modulation of Hsp90 function by ansamycins sensitizes breast cancer cells to chemotherapy-induced apoptosis in an RB- and schedule-dependent manner." Clin. Cancer Res. 7:2228-2236, 2001.

Murakami et al., "Induction of hsp 72/73 by herbimycin A, an inhibitor of transformation by tyrosine kinase oncogenes." Exp. Cell Res. 195:338-344, 1991.

Pratt and Toft, "Steroid receptor interactions with heat shock protein and immunophilin chaperones." Endocr. Rev. 18:306-60,1997.

Prodromou et al., "Identification and structural characterization of the ATP/ADP-binding site in the Hsp90 molecular chaperone." Cell 90:65-75, 1997.

Richter and Buchner, "Hsp90: chaperoning signal transduction." J. Cell. Physiol. 188:281-290, 2001.

Rosvold et al., "Identification of an NAD(P)H:quinone oxidoreductase polymorphism and its association with lung cancer and smoking." Pharmacogenetics 5:199-206, 1995.

Schneider et al., "Pharmacologic shifting of a balance between protein refolding and degradation mediated by Hsp90." Proc. Natl. Acad. Sci. USA 93:14536-14541, 1996.

Schnur et al., "erbB-2 oncogene inhibition by geldanamycin derivatives: synthesis, mechanism of action, and structure-activity relationships." J. Med. Chem. 38:3813-20, 1995.

Schnur et al., "Inhibition of the oncogene product p185erbB-2 in vitro and in vivo by geldanamycin and dihydrogeldanamycin derivatives." J. Med. Chem. 38:3806-3812, 1995.

Smith et al., "Progesterone receptor structure and function altered by geldanamycin, an hsp90-binding agent." Mol. Cell BioL 15:6804-6812, 1995.

Smith et al., "Identification of a 60-kilodalton stress-related protein, p60, which interacts with hsp90 and hsp70." Mol. Cell Biol. 13:869-876, 1993.

Stebbins et al., "Crystal structure of an Hsp90-geldanamycin complex: targeting of a protein chaperone by an antitumor agent." Cell 89:239-250,1997.

Traver et al., "NAD(P)H:quinone oxidoreductase gene expression in human colon carcinoma cells: characterization of a mutation which modulates DT-diaphorase activity and mitomycin sensitivity." Cancer Res. 52:797-802,1992.

Whitesell et al., "Inhibition of heat shock protein HSP90-pp60v-src heteroprotein complex formation by benzoquinone ansamycins: essential role for stress proteins in oncogenic transformation." Proc. Natl Acad. Sci. USA 91: 8324-8328, 1994.

Young et al., "Hsp90: a specialized but essential protein-folding tool." J. Cell Biol. 154:267-273, 2001.

Zou et al., "Repression of heat shock transcription factor HSF1 activation by HSP90 (HSP90 complex) that forms a stress-sensitive complex with HSF1." Cell 94:471-480, 1998.

*Discussion*

Geldanamycin (figure below, $R_{17}$ = $-OCH_3$) is a benzoquinone ansamycin polyketide isolated from *Streptomyces geldanus.* Although originally discovered by screening microbial extracts for antibacterial and antiviral activity, geldanamycin was later found to be cytotoxic to certain tumor cells *in vitro* and to reverse the morphology of cells transformed by the Rous sarcoma virus to a normal state.

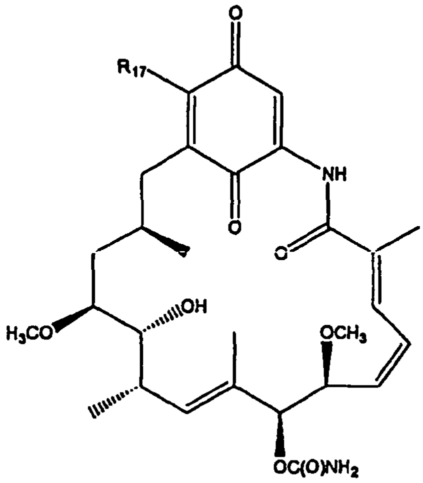

Geldanamycin's nanomolar potency and apparent specificity for aberrant protein kinase dependent tumor cells, as well as the discovery that its primary target in mammalian cells is the ubiquitous Hsp90 protein chaperone, has stimulated interest in the development of this compound as an anti-cancer drug. However, the association of unacceptable hepatotoxicity with the administration of geldanamycin led to its withdrawal from Phase I clinical trials.

More recently, attention has focused on 17-amino derivatives of geldanamycin, in particular 17-(allylamino)-17-desmethoxygeldanamycin ("17-AAG", $R_{17}$ = $-NCH_2CH=CH_2$). This compound has reduced hepatotoxicity while maintaining useful Hsp90 binding. Certain other 17-amino derivatives of geldanamycin, 11-oxogeldanamycin, and 5,6-dihydrogeldanamycin, are disclosed in U.S. patents 4,261,989, 5,387,584 and 5,932,566 Treatment of cancer cells with geldanamycin or 17-AAG causes a retinoblastoma protein-dependent G1 block, mediated by down-regulation of the induction pathways for cyclin D-cyclin dependent cdk4 and cdk6 protein kinase activity. Cell cycle arrest is followed by differentiation and apoptosis. G1 progression is unaffected by geldanamycin or 17-AAG in cells with mutated retinoblastoma protein; these cells undergo cell cycle arrest after mitosis, again followed by apoptosis.

The above-described mechanism of geldanamycin and 17-AAG appears to be a common mode of action among the benzoquinone ansamycins that further includes binding to Hsp90 and subsequent degradation of Hsp90-associated client proteins. Among the most sensitive client protein targets of the benzoquinone ansamycins are the Her kinases (also known as ErbB), Raf, Met tyrosine kinase, and the steroid receptors. Hsp90 is also involved in the cellular response to stress, including heat, radiation, and toxins. Certain benzoquinone ansamycins, such as 17-AAG, have thus been studied to determine their interaction with cytotoxins that do not target Hsp90 client proteins.

U.S. Patents 6,245,759, 6,306,874 and 6,313,138 disclose compositions comprising certain tyrosine kinase inhibitors together with 17-AAG and methods for treating cancer with such compositions. Münster, et al., "Modulation of Hsp90 function by ansamycins sensitizes breast cancer cells to chemotherapy induced apoptosis in an RB- and schedule-dependent manner," Clinical Cancer Research (2001) 7:2228-2236, discloses that 17-AAG sensitizes cells in culture to the cytotoxic effects of Paclitaxel and doxorubicin. The Münster reference further discloses that the sensitization towards paclitaxel by 17-AAG is schedule-dependent in retinoblastoma protein-producing cells due to the action of these two drugs at different stages of the cell cycle: treatment of cells with a combination of paclitaxel and 17-AAG is reported to give synergistic apoptosis, while pretreatment of cells with 17-AAG followed by treatment with paclitaxel is reported to result in abrogation of apoptosis. Treatment of cells with paclitaxel followed by treatment with 17-AAG 4 hours later is reported to show a synergistic effect similar to coincident treatment.

Citri, et al., "Drug-induced ubiquitylation and degradation of ErbB receptor tyrosine kinases: implications for cancer chemotherapy," EMBO Journal (2002) 21:2407-2417, discloses an additive effect upon co-administration of geldanamycin and an irreversible protein kinase inhibitor, CI-1033, on growth of ErbB2-expressing cancer cells in vitro. In contrast, an antagonistic effect of CI-1033 and anti-ErB2 antibody, Herceptin is disclosed.

Thus, while there has been a great deal of research interest in the benzoquinone ansamycins, particularly geldanamycin and 17-AAG, there remains a need for effective therapeutic regimens to treat cancer or other disease conditions characterized by undesired cellular hyperproliferation using such compounds, whether alone or in combination with other agents.

WO 03/013430 describes benzoquinone ansamycin analogs that may be useful in the treatment of cancer. WO 03/013430 suggests that these analogs may be used in combination with other agents, such as other drugs approved for the stand-alone treatment of cancer.

WO 02/36574 describes geldanamycin derivatives that can inhibit HSP90. These derivatives are proposed for use in the treatment of cancer, optionally in combination with other anticancer compounds.
WO 00/37050 describes drug delivery systems for the delivery of water insoluble drugs such as geldanamycin, 17-AAG and CA1. These drug delivery systems may be used in combination with other therapeutic methods such as the administration of other anticancer or antitumor compounds.

## SUMMARY OF THE INVENTION

The present invention relates to a method for treating cancer. The method involves the administration of an HSP90 inhibitor that is 17-AAG and an antimetabolite that is gemcitabine, where the combined administration provides a synergistic effect.
The subject may be treated with a dose of an HSP90 inhibitor in one step and a dose of an antimetabolite in another step. The invention relates to a method for treating colorectal cancer where a subject is treated with a dose of HSP90 inhibitor in one step and a dose of an antimetabolite in another step. The HSP90 inhibitor is 17-AAG, while the antimetabolite is gemcitabine. The gemcitabine is administered after the 17-AAG. Thus, the present invention provides the use of an HSP90 inhibitor that is 17-AAG and an antimetabolite that is gemcitabine for the manufacture of medicaments for treating colorectal cancer in a patient, wherein the 17-AAG is administered to the patient before the gemcitabine.
A method of treating cancer is described herein where a subject is first treated with a dose of an antimetabolite (e.g. 5-fluorouracil or gemcitabine). After waiting for a period of time sufficient to allow development of a substantially efficacious response of the antimetabolite, a formulation comprising a synergistic dose of a benzoquinone ansamycin together with a second sub-toxic dose of the antimetabolite is administered.
A method of treating cancer is described herein where a subject is treated first with a dose of a benzoquinone ansamycin, and second, a dose of an antimetabolite. After waiting for a period of time sufficient to allow development of a substantially efficacious response of the antimetabolite, a formulation comprising a synergistic dose of a benzoquinone ansamycin together with a second sub-toxic dose of the oncolytic drug is administered.
A method for treating cancer is described herein where a subject is treated with a dose of an HSP90 inhibitor in one step and a dose of an antimetabolite in another step, and where a side effect profile for the combined, administered drugs is substantially better than for the antimetabolite alone.

### Definitions

"Antimetabolite" refers to an antineoplastic drug that inhibits the utilization of a metabolite or a prodrug thereof. Examples of antimetabolites include, without limitation, methotrexate, 5-fluorouracil, 5-fluorouracil prodrugs (e.g. capecitabine), 5-fluorodeoxyuridine monophosphate, cytarabine, 5-azacytidine, gemcitabine, mercaptopurine, thioguanine, azathioprine, adenosine, pentostatin, erythrohydroxynonyladenine, and cladribine.
"HSP90 inhibitor" refers to a compound that inhibits the activity of heat shock protein 90, which is a cellular protein responsible for chaperoning multiple client proteins necessary for cell signalling, proliferation and survival. One class of HSP90 inhibitors is the benzoquinone ansamycins. Examples of such compounds include, without limitation, geldanamycin and geldanamycin derivatives (e.g. 17-alkylamino-17-desmethoxy-geldanamycin ("17-AAG") and 17-(2 dimethylaminoethyl)amino-17-desmethoxy-geldanamycin ("17-DMAG")). See Sasaki *et al.,* US 4,261,989 (1981) for synthesis of 17-AAG and *Snader et al.,* US 2004/0053909 A1 (2004) for synthesis of 17-DMAG. In addition to 17-AAG and 17-DMAG, other preferred geldanamycin derivatives are 11-*O*-methyl-17-(2-(1-azetidinyl)ethyl)amino-17-demethoxygeldanamycin (A), 11-*O*-methyl-17-(2-dimethylaminoethyl)amino-17-demethoxygeldanamycin (B), and 11-*O*-methyl-17-(2-(1 pyrrolidinyl)ethyl)amino-17-demethoxygeldanamycin (C), whose synthesis is described in the co-pending commonly US patent application of Tian *et al.,* serial no. 10/825,788, filed Apr.16, 2004, and in Tian *et al.,* PCT application no. PCT/US04/11638, filed Apr. 16, 2004. Additional preferred geldanamycin derivatives are described in Santi *et al.,* US 2003/0114450 A1 (2003).

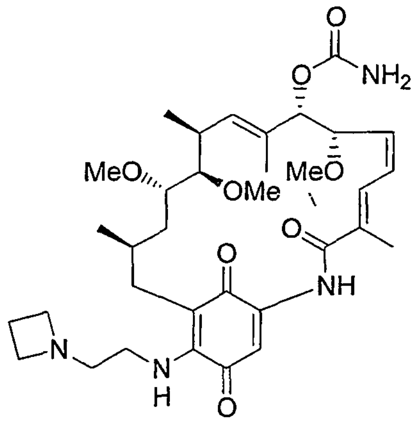

(A)

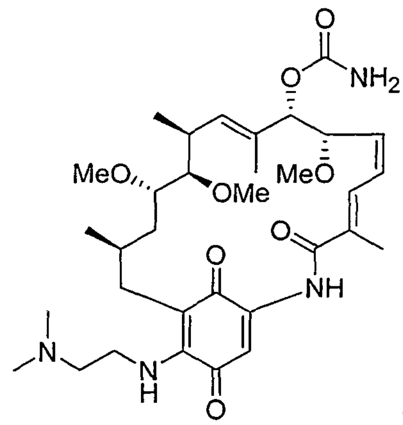

(B)

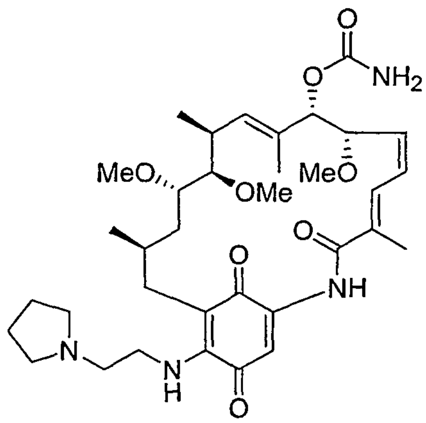

(C)

"MTD" refers to maximum tolerated dose. The MTD for a compound is determined using methods and materials known in the medical and pharmacological arts, for example through dose-escalation experiments. One or more patients is first treated with a low dose of the compound, typically about 10% of the dose anticipated to be therapeutic based on results of in vitro cell culture experiments. The patients are observed for a period of time to determine the occurrence of toxicity. Toxicity is typically evidenced as the observation of one or more of the following symptoms: vomiting, diarrhea, peripheral neuropathy, ataxia, neutropenia, or elevation of liver enzymes. If no toxicity is observed, the dose is increased about 2-fold, and the patients are again observed for evidence of toxicity. This cycle is repeated until a dose producing evidence of toxicity is eached. The dose immediately preceding the onset of unacceptable toxicity is taken as the MTD.

"Side effects" refer to a number of toxicities typically seen upon treatment of a subject with an antineoplastic drug. Such toxicities include, without limitation, anemia, anorexia, bilirubin effects, dehydration, dermatology effects, diarrhea, dizziness, dyspnea, edema, fatigue, headache, hematemesis, hypokalemia, hypoxia, musculoskeletal effects, myalgia, nausea, neuro-sensory effects, pain, rash, serum glutamic oxaloacetic transaminase effects, serum glutamic pyruvic transaminase effects, stomatitis, sweating, taste effects, thrombocytopenia, voice change, and vomiting.

"Side effect grading" refers to National Cancer Institute common toxicity criteria (NCI CTC, Version 2). Grading runs from 1 to 4, with a grade of 4 representing the most serious toxicities.

<u>Combination Therapy</u>

The present invention relates to a method for treating cancer. The method involves the administration of an HSP90 inhibitor that is 17-AAG and an antimetabolite that is gemcitabine, where the combined administration provides a synergistic effect.

HSP90 inhibitors include benzoquinone ansamycins. A typical benzoquinone ansamycin is geldanamycin or a geldanamycin derivative. A benzoquinone ansamycin may be a geldanamycin derivative selected from a group consisting of 17-alkylamino-17-desmethoxy-geldanamycin ("17-AAG"), 17-(2-dimethylaminoethyl)amino-17-desmethoxy-geldanamycin ("17-DMAG"), 11-*O*-methyl-17-(2-(1-azetidinyl)ethyl)amino-17-demethoxygeldanamycin, 11-*O*-methyl-17-(2-dimethylaminoethyl)amino-17-demethoxygeldanamycin, and 11-*O*-methyl-17-(2-(1-pyrrolidinyl)ethyl)amino-17-demethoxygeldanamycin.

Antimetabolites include, without limitation, methotrexate, 5-fluorouracil, 5-fluorouracil prodrugs (e.g., capecitabine), 5-fluorodeoxyuridine monophosphate, cytarabine, 5-azacytidine, gemcitabine, mercaptopurine, thioguanine, azathioprine, adenosine, pentostatin, erythrohydroxynonyladenine, and cladribine. The antimetabolite used in accordance with the invention is gemcitabine.

The dose of antimetabolite used as a partner in combination therapy with an HSP90 inhibitor (e.g., benzoquinone ansamycin) is determined based on the maximum tolerated dose observed when the antimetabolite is used as the sole therapeutic agent. In one embodiment of the invention, the dose of antimetabolite when used in combination therapy with a benzoquinone ansamycin is the MTD. In other embodiments of the invention, the dose of antimetabolite when used in combination therapy with a benzoquinone ansamycin is between about 1% of the MTD and the MTD, between about 5% of the MTD and the MTD, between about 5% of the MTD and 75% of the MTD, or between about 25% of the MTD and 75% of the MTD.

Use of the benzoquinone ansamycin allows for use of a lower therapeutic dose of an antimetabolite, thus significantly widening the therapeutic window for treatment. In one embodiment, the therapeutic dose of antimetabolite is lowered by at least about 10%. In other embodiments the therapeutic dose is lowered from about 10 % to 20%, from about 20% to 50%, from about 50% to 200%, or from about 100% to 1,000%.

For the treatment of a variety of carcinomas, the recommended dose of the antimetabolite 5-fluorouracil for an average patient is 12 mg/kg daily for 4 days, by rapid injection, followed by 6 mg/kg on alternate succeeding days for four doses if no toxicity is observed. An arbitrary maximal daily dose for the preceding regimen has been set at 800 mg. Other regimens use daily doses of 500 mg/m$^2$ 5-fluorouracil for 5 days, repeated in monthly cycles. For the antimetabolite gemcitabine, the administered dose is typically 1,000 to 1,500 mg/m$^2$ over 30 min once a week.

The synergistic dose of the benzoquinone ansamycin used in combination therapy is determined based on the maximum tolerated dose observed when the benzoquinone ansamycin is used as the sole therapeutic agent. Clinical trials have determined an MTD for 17-AAG of about 40 mg/m$^2$ utilizing a daily x 5 schedule, an MTD of about 220 mg/m$^2$ utilizing a twice-weekly regimen, and an MTD of about 308 mg/m$^2$ utilizing a once-weekly regimen. The dose of the benzoquinone ansamycin when used in combination therapy may be the MTD. The dose of the benzoquinone ansamycin when used in combination therapy may be between about 1% of the MTD and the MTD, between about 5% of the MTD and the MTD, between about 5% of the MTD and 75% of the MTD, or between about 25% of the MTD and 75% of the MTD.

Where the benzoquinone ansamycin is 17-AAG, and the administration of compound is weekly, its therapeutic dose is typically between 50 mg/m$^2$ and 450 mg/m$^2$. Preferably, the dose is between 150 mg/m$^2$ and 350 mg/m$^2$, and about 308 mg/m$^2$ is especially preferred. Where the administration of compound is biweekly (i. e., twice per week), the therapeutic dose of 17-AAG is typically between 50 mg/m$^2$ and 250 mg/m$^2$. Preferably, the dose is between 150 mg/m$^2$ and 250 mg/m$^2$, and about 220 mg/m$^2$ is especially preferred.

Where the present method involves the administration of 17-AAG and gemcitabine, a dosage regimen involving one or two administrations of the combination per week is typical. Tables 3 and 4 below show a number of gemcitabine/17-AAG dosage combinations (i.e., dosage combinations 0097 to 0212).

**Table 3: Gemcitabine/17-AAG dosage combinations.**

| | **30-100 mg/m$^2$ 17-AAG** | **100-150 mg/m$^2$ 17-AAG** | **150-200 mg/m$^2$ 17-AAG** | **200-250 mg/m$^2$ 17-AAG** |
|---|---|---|---|---|
| **0-100 mg/m$^2$ gemcitabine** | 0097 | 0098 | 0099 | 0100 |
| **100-200 mg/m$^2$ gemcitabine** | 0101 | 0102 | 0103 | 0104 |
| **200-300 mg/m$^2$ gemcitabine** | 0105 | 0106 | 0107 | 0108 |
| **300-400 mg/m$^2$ gemcitabine** | 0109 | 0110 | 0111 | 0112 |
| **400-500 mg/m$^2$ gemcitabine** | 0113 | 0114 | 0115 | 0116 |
| **500-600 mg/m$^2$ gemcitabine** | 0117 | 0118 | 0119 | 0120 |

(continued)

| | 30-100 mg/m$^2$ 17-AAG | 100-150 mg/m$^2$ 17-AAG | 150-200 mg/m$^2$ 17-AAG | 200-250 mg/m$^2$ 17-AAG |
|---|---|---|---|---|
| **600-700 mg/m$^2$ gemcitabine** | 0121 | 0122 | 0123 | 0124 |
| **700-800 mg/m$^2$ gemcitabine** | 0125 | 0126 | 0127 | 0128 |
| **- 800-900 mg/m$^2$ gemcitabine** | 0129 | 0130 | 0131 | 0132 |
| **900-1000 mg/m$^2$ gemcitabine** | 0133 | 0134 | 0135 | 0136 |
| **1000-1100 mg/m$^2$ gemcitabine** | 0137 | 0138 | 0139 | 0140 |
| **1100-1200 mg/m$^2$ gemcitabine** | 0141 | 0142 | 0143 | 0144 |
| **1200-1300 mg/m$^2$ gemcitabine** | 0145 | 0146 | 0147 | 0148 |
| **1300-1400 mg/m$^2$ gemcitabine** | 0149 | 0150 | 0151 | 0152 |
| **1400-1500 mg/m$^2$ gemcitabine** | 0153 | 0154 | 0155 | 0156 |

**Table 4: Gemcitabine/17-AAG dosage combinations continued.**

| | 250-300 mg/m$^2$ 17-AAG | 300-350 mg/m$^2$ 17-AAG | 350-400 mg/kg 17-AAG | 400-450 mg/kg 17-AAG |
|---|---|---|---|---|
| **0-100 mg/m$^2$ gemcitabine** | 0153 | 0154 | 0155 | 0156 |
| **100-200 mg/m$^2$ gemcitabine** | 0157 | 0158 | 0159 | 0160 |
| **200-300 mg/m$^2$ gemcitabine** | 0161 | 0162 | 0163 | 0164 |
| **300-400 mg/m$^2$ gemcitabine** | 0165 | 0166 | 0167 | 0168 |
| **400-500 mg/m$^2$ gemcitabine** | 0169 | 0170 | 0171 | 0172 |
| **500-600 mg/m$^2$ gemcitabine** | 0173 | 0174 | 0175 | 0176 |
| **600-700 mg/m$^2$ gemcitabine** | 0177 | 0178 | 0179 | 0180 |
| **700-800 mg/m$^2$ gemcitabine** | 0181 | 0182 | 0183 | 0184 |
| **800-900 mg/m$^2$ gemcitabine** | 0185 | 0186 | 0187 | 0188 |
| **900-1000 mg/m$^2$ gemcitabine** | 0189 | 0190 | 0191 | 0192 |

(continued)

| | 250-300 mg/m$^2$ 17-AAG | 300-350 mg/m$^2$ 17-AAG | 350-400 mg/kg 17-AAG | 400-450 mg/kg 17-AAG |
|---|---|---|---|---|
| **1000-1100 mg/m$^2$ gemcitabine** | 0193 | 0194 | 0195 | 0196 |
| **1100-1200 mg/m$^2$ gemcitabine** | 0197 | 0198 | 0199 | 0200 |
| **1200-1300 mg/m$^2$ gemcitabine** | 0201 | 0202 | 0203 | 0204 |
| **1300-1400 mg/m$^2$ gemcitabine** | 0205 | 0206 | 0207 | 0208 |
| **1400-1500 mg/m$^2$ gemcitabine** | 0209 | 0210 | 0211 | 0212 |

A subject is first treated with a dose of an HSP90 inhibitor and subsequently treated with a dose of an antimetabolite.

A subject may be first treated with a dose of an antimetabolite (e.g., 5-fluorouracil or gemcitabine). After waiting for a period of time sufficient to allow development of a substantially efficacious response of the oncolytic drug, a formulation comprising a synergistic dose of a benzoquinone ansamycin together with a second sub-toxic dose of the antimetabolite may be administered. In general, the appropriate period of time sufficient to allow development of a substantially efficacious response to the antimetabolite will depend upon the pharmacokinetics of the antimetabolite, and will have been determined during clinical trials of therapy using the antimetabolite alone. The period of time sufficient to allow development of a substantially efficacious response to the antimetabolite may be between about 1 hour and 96 hours. The period of time sufficient to allow development of a substantially efficacious response to the antimetabolite may be between about 2 hours and 48 hours. The period of time sufficient to allow development of a substantially efficacious response to the antimetabolite may be between about 4 hours and 24 hours.

A subject may be treated first with one of the above-described benzoquinone ansamycins, and second, a dose of an antimetabolite, such as, but not limited to, 5-fluorouracil and gemcitabine. After waiting for a period of time sufficient to allow development of a substantially efficacious response of the antimetabolite, a formulation comprising a synergistic dose of a benzoquinone ansamycin together with a second sub-toxic dose of the antimetabolite may be administered. In general, the appropriate period of time sufficient to allow development of a substantially efficacious response to the antimetabolite will depend upon the pharmacokinetics of the antimetabolite, and will have been determined during clinical trials of therapy using the antimetabolite alone. The period of time sufficient to allow development of a substantially efficacious reponse to the antimetabolite may be between about 1 hour and 96 hours. The period of time sufficient to allow development of a substantially efficacious response to the antimetabolite may be between about 2 hours and 48 hours. The period of time sufficient to allow development of a substantially efficacious response to the antimetabolite may be between about 4 hours and 24 hours.

As noted above, the combination of an HSP90 inhibitor and an antimetabolite allows for the use of a lower therapeutic dose of the antimetabolite for the treatment of cancer. That a lower dose of antimetabolite is used oftentimes lessens the side effects observed in a subject. The lessened side effects can be measured both in terms of incidence and severity. Severity measures are provided through a grading process delineated by the National Cancer Institute (common toxicity criteria NCI CTC, Version 2). For instance, the incidence of side effects are typically reduced 10%. Oftentimes, the incidence is reduced 20%, 30%, 40% or 50%. Furthermore, the incidence of grade 3 or 4 toxicities for more common side effects associated with antimetabolite administration (e.g., anemia, anorexia, diarrhea, fatigue, nausea and vomiting) is oftentimes reduced 10%, 20%, 30%, 40% or 50%.

Formulations used in the present invention may be in any suitable form, such as a solid, semisolid, or liquid form. See *Pharmaceutical Dosage Forms and Drug Delivery Systems,* 5$^{th}$ edition, Lippicott Williams & Wilkins (1991). In general the pharmaceutical preparation will contain one or more of the compounds described herein as an active ingredient in admixture with an organic or inorganic carrier or excipient suitable for external, enteral, or parenteral application. The active ingredient may be compounded, for example, with the usual non-toxic, pharmaceutically acceptable carriers for tablets, pellets, capsules, suppositories, pessaries, solutions, emulsions, suspensions, and any other form suitable for use. The carriers that can be used include water, glucose, lactose, gum acacia, gelatin, mannitol, starch paste, magnesium trisilicate, talc, corn starch, keratin, colloidal silica, potato starch, urea, and other carriers suitable for use in manufacturing preparations in solid, semi-solid, or liquefied form. In addition, auxiliary stabilizing, thickening, and

coloring agents and perfumes may be used. Where applicable, the compounds useful in the invention may be formulated as microcapsules and nanoparticles. General protocols are described, for example, by Microcapsules and Nanoparticles in Medicine and Pharmacy by Max Donbrow, ed., CRC Press (1992) and by U.S. Patent Nos. 5,510,118, 5,534,270 and 5,662,883 which are all incorporated herein by reference. By increasing the ratio of surface area to volume, these formulations allow for the oral delivery of compounds that would not otherwise be amenable to oral delivery. The compounds useful in the invention may also be formulated using other methods that have been previously used for low solubility drugs. For example, the compounds may form emulsions with vitamin E or a PEGylated derivative thereof as described by PCT publications WO 98/30205 and WO 00/71163. Typically, the compound useful in the invention is dissolved in an aqueous solution containing ethanol (preferably less than 1% w/v). Vitamin E or a PEGylated-vitamin E is added. The ethanol is then removed to form a pre-emulsion that can be formulated for intravenous or oral routes of administration. Another method involves encapsulating the compounds useful in the invention in liposomes. Methods for forming liposomes as drug delivery vehicles are well known in the art. Suitable protocols include those described by U.S. Patent Nos. 5,683,715, 5,415,869, and 5,424,073 relating to another relatively low solubility cancer drug paclitaxel and by PCT Publicaton WO 01/10412 relating to epothilone B. Of the various lipids that may be used, particularly preferred lipids for making encapsulated liposomes include phosphatidylcholine and polyethyleneglycol-derivatized distearyl phosphatidylethanoloamine.

Yet another method involves formulating the compounds useful in the invention using polymers such as biopolymers or biocompatible (synthetic or naturally occurring) polymers. Biocompatible polymers can be categorized as biodegradable and non-biodegradable. Biodegradable polymers degrade in vivo as a function of chemical composition, method of manufacture, and implant structure. Illustrative examples of synthetic polymers include polyanhydrides, polyhydroxyacids such as polylactic acid, polyglycolic acids and copolymers thereof, polysters, polyamides, polyorthoesters and some polyphosphazenes. Illustrative examples of naturally occurring polymers include proteins and polysaccharides such as collagen, hyaluronic acid, albumin, and gelatin.

Another method involves conjugating the compounds useful in the invention to a polymer that enhances aqueous solubility. Examples of suitable polymers include polyethylene glycol, poly-(d-glutamic acid), poly-(1-glutamic acid), poly-(1-glutamic acid), poly-(d-aspartic acid), poly-(1-aspartic acid) and copolymers thereof. Polyglutamic acids having molecular weights between about 5,000 to about 100,000 are preferred, with molecular weights between about 20,000 and 80,000 being more preferred wand with molecular weights between about 30,000 and 60,000 being most preferred. The polymer is conjugated via an ester linkage to one or more hydroxyls of an inventive geldanamycin using a protocol as essentially described by U.S. Patent No. 5,977,163.

In another method, the compounds useful in the invention are conjugated to a monoclonal antibody. This method allows the targeting of the inventive compounds to specific targets. General protocols for the design and use of conjugated antibodies are described in Monoclonal Antibody-Based Therapy of Cancer by Michael L. Grossbard, ED. (1998).

The amount of active ingredient that may be combined with the carrier materials to produce a single dosage form will vary depending upon the subject treated and the particular mode of administration. For example, a formulation for intravenous use comprises an amount of the inventive compound ranging from about 1 mg/mL to about 25 mg/mL, preferably from about 5 mg/mL, and more preferably about 10 mg/mL. Intravenous formulations are typically diluted between about 2 fold and about 30 fold with normal saline or 5% dextrose solution prior to use.

Preferably, 17-AAG is formulated as a pharmaceutical solution formulation comprising 17-AAG in an concentration of up to 15 mg/mL dissolved in a vehicle comprising (i) a first component that is ethanol, in an amount of between about 40 and about 60 volume %; (ii) a second component that is a polyethoxylated castor oil, in an amount of between about 15 to about 50 volume %; and (iii) a third component that is selected from the group consisting ofpropylene glycol, PEG 300, PEG 400, glycerol, and combinations thereof, in an amount of between about 0 and about 35 volume %. The aforesaid percentages are volume/volume percentages based on the combined volumes of the first, second, and third components. The lower limit of about 0 volume % for the third component means that it is an optional component; that is, it may be absent. The pharmaceutical solution formulation is then diluted into water to prepare a diluted formulation containing up to 3 mg/mL 17-AAG, for intravenous formulation.

Preferably, the second component is Cremophor EL and the third component is propylene glycol. In an especially preferred formulation, the percentages of the first, second, and third components are 50%, 20-30%, and 20-30%, respectively.

Other formulations designed for 17-AAG are described in Tabibi et al., US 6,682,758 B1 (2004) and Ulm et al., WO 03/086381 A1 (2003).

The present invention is used for the treatment of cancer. The methods described herein may be used to treat cancers of the head and neck, which include, but are not limited to, tumors of the nasal cavity, paranasal sinuses, nasopharynx, oral cavity, oropharynx, larynx, hypopharynx, salivary glands, and paragangliomas. The compounds described herein may be used to treat cancers of the liver and biliary tree, particularly hepatocellular carcinoma. The compounds described herein may be used to treat intestinal cancers, particularly colorectal cancer. The compounds

described herein may be used to treat ovarian cancer. The compounds described herein may be used to treat small cell and non-small cell lung cancer. The compounds described herein may be used to treat breast cancer. The compounds described herein may be used to treat sarcomas, including fibrosarcoma, malignant fibrous histiocytoma, embryonal rhabdomysocarcoman, leiomysosarcoma, neuro-fibrosarcoma, osteosarcoma, synovial sarcoma, liposarcoma, and alveolar soft part sarcoma. The compounds described herein may be used to treat neoplasms of the central nervous systems, particularly brain cancer. The compounds described herein may be used to treat lymphomas which include Hodgkin's lymphoma, lymphoplasmacytoid lymphoma, follicular lymphoma, mucosa-associated lymphoid tissue lymphoma, mantle cell lymphoma, B-lineage large cell lymphoma, Burkitt's lymphoma, and T-cell anaplastic large cell lymphoma.

## Examples

The following Examples are provided to illustrate certain aspects of the present invention and to aid those of skill in the art in practicing the invention.

## Materials and Methods

### *Cell line and reagents*

Human colon adenocarcinoma cell line, DLD-1, and human breast adenocarcinoma cell line, SKBr-3, were obtained from American Type Culture Collection (manassas, VA). DLD-1 cells were maintained in RPMI 1640 medium supplemented with 10% fetal bovine serum, and SKBr-3 cells were cultured in McCoy's 5a medium supplemented with 10 % fetal bovine serum. 17-DMAG and 17-AAG were obtained using published procedures. Other cytotoxic agents were purchased commercially from suppliers such as Sigma Chemical Co. (St. Louis, MO) and Sequoia Research Products (Oxford, UK).

### *Cell viability assay and combination effect analysis*

Cells were seeded in duplicate in 96-well microtiter plates at a density of 5,000 cells per well and allowed to attach overnight. Cells were treated with 17-AAG or 17-DMAG and the corresponding cytotoxic drug at varying concentrations, ranging from 0.5 picomolar ("pM") to 50 micromolar ("μM"), for 3 days. Cell viability was determined using the MTS assay (Promega). For the drug combination assay, cells were seeded in duplicate in 96-well plates (5,000 cells/well). After an overnight incubation, cells were treated with drug alone or a combination and the $IC_{50}$ value (the concentration of drug required to inhibit cell growth by 50%) was determined. Based on the $IC_{50}$ values of each individual drug, combined drug treatment was designed at constant ratios of two drugs, i.e., equivalent to the ratio of their $IC_{50}$. Two treatment schedules were used: In one schedule, the cells were exposed to 24 hours of 17-AAG or 17-DMAG. The drug was then added to the cells and incubated for 48 hours. In another schedule, cells were exposed to the drug alone for 24 hours followed by addition of 17-AAG or 17-DMAG for 48 hours. Cell viability was determined by the MTS assay.

Synergism, additivity or antagonism was determined by median effect analysis using the combination index (CI) calculated using Calcusyn (Biosoft, Cambridge, UK). The combination index is defined as follows:

$$CI = [D]_1/[D_x]_1 + [D]_2/[D_x]_2$$

The quantities $[D]_1$ and $[D]_2$ represent the concentrations of the first and second drug, respectively, that in combination provide a response of x % in the assay. The quantities $[D_x]_1$ and $[D_x]_2$ represent the concentrations of the first and second drug, respectively, that when used alone provide a response of x % in the assay. Values of CI < 1, CI = 1, and CI > 1 indicated drug-drug synergism, additivity, and antagonism respectively (Chou and Talalay 1984). The "enhancing" effect of two drugs can also be determined.

## Results

### *17-AAG combination in DLD-1 cells*

The following table provides CI values for combinations of 17-AAG and the antimetabolites 5-fluorouracil, gemicitabine and methotrexate in a DLD-1 cell assay. "Pre-administration" refers to the administration of 17-AAG to the cells before the administration of antimetabolite; "post-administration" refers to the administration of 17-AAG to the cells

after the administration of antimetabolite.

**Table 5: CI values for combinations in DLD-1 cells (human colorectal cancer cells).**

| Antimetabolite | 17-AAG Pre-Administration | 17-AAG Post-Administration |
|---|---|---|
| 5-Fluorouracil | 0.96 ± 0.16 | 0.33 ± 0.08 |
| Gemcitabine | 0.45 ± 0.19 | 0.77 ± 0.23 |
| Methotrexate | 0.83 ± 0.14 | 0.71 ± 0.35 |

*17-AAG combination in SKSBr-3 cells*

The following table provides CI values for combinations of 17-AAG and the antimetabolites 5-fluorouracil and gemicitabine in an SKBr-3 cell assay.

**Table 6: CI values for combinations in SKBr cells (human breast cancer cells).**

| Antimetabolite | 17-AAG Pre-Administration | 17-AAG Post-Administration |
|---|---|---|
| 5-Fluorouracil | 0.65 ± 0.48 | 0.1.01 ± 0.13 |
| Gemcitabine | 1.79 ± 0.54 | 0.86 ± 0.19 |

*Additional Observations*

Additional analysis indicated that both 17-AAG and 17-DMAG reduced the expression of ErbB2 protein in SKBr3 and glioma cells. This observation, taken in combination with the results reported above, indicates that combinations of 17-AAG or 17-DMAG with any of the antimetabolites above that are known to be useful to treat diseases characterized by elevated ErbB2 protein expression (i.e., levels of expressions of ErbB2 protein greater than those found in healthy cells). Similarly, combinations of 17-AAG and 5-FU reduced the expression ofRaf-1 and Src kinase proteins, also demonstrating that this combination is especially effective in treating diseases characterized by elevated expression of these two proteins.

## Claims

**1.** Use of an HSP90 inhibitor that is 17-AAG and an antimetabolite that is gemcitabine for the manufacture of medicaments for treating colorectal cancer in a patient, wherein the 17-AAG is administered to the patient before the gemcitabine.

**2.** The use of claim 1, wherein the therapeutic dose of gemcitabine is 1000 to 1500 mg/$m^2$ over 30 min once a week.

**3.** The use of claim 1, wherein the administration of 17-AAG and the gemcitabine is performed once per week.

**4.** The use of claim 3, wherein the therapeutic dose of 17-AAG is between 50 mg/$m^2$ and 450 mg/$m^2$.

**5.** The use of claim 3, wherein the therapeutic dose of 17-AAG is between 150 mg/$m^2$ and 350 mg/$m^2$.

**6.** The use of claim 3, wherein the therapeutic dose of 17-AAG is about 308 mg/$m^2$.

**7.** The use of claim 1, wherein the administration of 17-AAG and the gemcitabine is performed twice per week.

**8.** The use of claim 7, wherein the therapeutic dose of 17-AAG is between 50 mg/$m^2$ and 250 mg/$m^2$.

**9.** The use of claim 7, wherein the therapeutic dose of 17-AAG is between 150 mg/$m^2$ and 250 mg/$m^2$.

**10.** The use of claim 7, wherein the therapeutic dose of 17-AAG is about 220 mg/$m^2$.

## Patentansprüche

1. Verwendung eines HSP90-Inhibitors, der 17-AAG ist, und eines Antimetaboliten, der Gemcitabin ist, für die Herstellung von Medikamenten zur Behandlung von kolorektalem Krebs bei einem Patienten, wobei das 17-AAG dem Patienten vor dem Gemcitabin verabreicht wird.

2. Verwendung nach Anspruch 1, wobei die therapeutische Dosis an Gemcitabin 1000 bis 1500 $mg/m^2$ über 30 Min. einmal wöchentlich beträgt.

3. Verwendung nach Anspruch 1, wobei die Verabreichung von 17-AAG und des Gemcitabins einmal wöchentlich erfolgt.

4. Verwendung nach Anspruch 3, wobei die therapeutische Dosis von 17-AAG zwischen 50 $mg/m^2$ und 450 $mg/m^2$ beträgt.

5. Verwendung nach Anspruch 3, wobei die therapeutische Dosis von 17-AAG zwischen 150 $mg/m^2$ und 350 $mg/m^2$ beträgt.

6. Verwendung nach Anspruch 3, wobei die therapeutische Dosis von 17-AAG etwa 308 $mg/m^2$ beträgt.

7. Verwendung nach Anspruch 1, wobei die Verabreichung von 17-AAG und des Gemcitabins zweimal wöchentlich erfolgt.

8. Verwendung nach Anspruch 7, wobei die therapeutische Dosis von 17-AAG zwischen 50 $mg/m^2$ und 250 $mg/m^2$ beträgt.

9. Verwendung nach Anspruch 7, wobei die therapeutische Dosis von 17-AAG zwischen 150 $mg/m^2$ und 250 $mg/m^2$ beträgt.

10. Verwendung nach Anspruch 7, wobei die therapeutische Dosis von 17-AAG etwa 220 $mg/m^2$ beträgt.

## Revendications

1. Utilisation d'un inhibiteur de HSP90 qui est le 17-AAG et d'un antimétabolite qui est la gemcitabine pour la fabrication de médicaments afin de traiter le cancer colorectal chez un patient, dans laquelle le 17-AAG est administré au patient avant la gemcitabine.

2. Utilisation selon la revendication 1, dans laquelle la dose thérapeutique de gemcitabine est de 1 000 à 1 500 $mg/m^2$ sur 30 min une fois par semaine.

3. Utilisation selon la revendication 1, dans laquelle l'administration du 17-AAG et de la gemcitabine est effectuée une fois par semaine.

4. Utilisation selon la revendication 3, dans laquelle la dose thérapeutique du 17-AAG se situe entre 50 $mg/m^2$ et 450 $mg/m^2$.

5. Utilisation selon la revendication 3, dans laquelle la dose thérapeutique du 17-AAG se situe entre 150 $mg/m^2$ et 350 $mg/m^2$.

6. Utilisation selon la revendication 3, dans laquelle la dose thérapeutique du 17-AAG est aux alentours de 308 $mg/m^2$.

7. Utilisation selon la revendication 1, dans laquelle l'administration du 17-AAG et de la gemcitabine est effectuée deux fois par semaine.

8. Utilisation selon la revendication 7, dans laquelle la dose thérapeutique du 17-AAG se situe entre 50 $mg/m^2$ et 250 $mg/m^2$.

**9.** Utilisation selon la revendication 7, dans laquelle la dose thérapeutique du 17-AAG se situe entre 150 $mg/m^2$ et 250 $mg/m^2$.

**10.** Utilisation selon la revendication 7, dans laquelle la dose thérapeutique du 17-AAG est aux alentours de 220 $mg/m^2$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6245759 B
- US 6306874 B
- US 6313138 B
- US 4261989 A
- US 5387584 A
- US 5932566 A
- WO 03013430 A
- WO 0236574 A
- WO 0037050 A
- US 20040053909 A1
- US 0411638 W
- US 20030114450 A1
- US 5510118 A
- US 5534270 A
- US 5662883 A
- WO 9830205 A
- WO 0071163 A
- US 5683715 A
- US 5415869 A
- US 5424073 A
- WO 0110412 A
- US 5977163 A
- US 6682758 B1
- WO 03086381 A1


### Non-patent literature cited in the description


- **AGNEW et al.** Clinical pharmacokinetics of 17-(allylamino)-17-demethoxygeldanamycin and the active metabolite 17-(amino)-17-demethoxygctdanamycin given as a one-hour infusion daily for 5 days. *AACR,* 2002
- **AN et al.** Depletion of p185erbB2, Raf-1 and mutant p53 proteins by geldanamycin derivatives correlates with antiproliferative activity. *Cancer Chemother. Pharmacol.,* 1997, vol. 40, 60-64
- **BAGATELL et al.** Induction of a heat shock factor 1-dependent stress response alters the cytotoxic activity of hsp90-binding agents. *Clin. Cancer Res.,* 2000, vol. 6, 3312-3318
- **BAGATELL et al.** Destabilization of steroid receptors by heat shock protein 90-binding drugs: a ligand-independent approach to hormonal therapy of breast cancer. *Clin. Cancer Res.,* 2001, vol. 7, 2076-2084
- **BANERJI et al.** A pharmacokinetically (PK)-pharmacodynamically (PD) driven Phase I trial of the HSP90 molecular chaperone inhibitor 17-allylamino-17-demethoxygeldanamycin (17-AAG). *AACR,* 2002
- **BARENT et al.** Analysis of FKBP51/FKBP52 chimeras and mutants for Hsp90 binding and association with progesterone receptor complexes. *Mol. Endocrinol.,* 1998, vol. 12, 342-354
- **BILODEAU et al.** *Tyrosine kinase inhibitors.*
- **CITRI et al.** Drug-induced ubiquitylation and degradation of ErbB receptor tyrosine dinases: implications for cancer chemotherapy. *EMBO Journal,* 2002, vol. 21, 2407-2417
- **EGORIN et al.** Metabolism of 17-(allylamino)-17-demethoxygeldanamycin (NSC 330507) by murine and human hepatic preparations. *Cancer Res.,* 1998, vol. 58, 2385-2396
- **FRALEY et al.** *Tyrosine kinase inhibitors.*
- **GAIDIGK.** NAD(P)H:quinone oxidoreductase: polymorphisms and allele frequencies n Caucasian, Chinese and Canadian Native Indian and Inuit populations. *Pharmacogenetics,* 1998, vol. 8, 305-313
- **GELMON et al.** Anticancer agents targeting signaling molecules and cancer cell environment: challenges for drug development?. *J. Natl. Cancer Inst.,* 1999, vol. 91, 128 1-1287
- **GOETZ et al.** The Hsp90 chaperone complex as a novel target for cancer therapy. *Ann. Oncol.,* 2003, vol. 14, 1169-1176
- **GOH et al.** Explaining interindividual variability of docetaxel pharmacokinetics and pharmacodynamics in Asians through phenotyping and genotyping strategies. *J. Clin. Oncol.,* 2002, vol. 20, 3683-3690
- **GRENERT et al.** The amino-terminal domain of heat shock protein 90 (hsp90) that binds geldanamycin is an ATP/ADP switch domain that regulates hsp90 conformation. *J. Biol. Chem.,* 1997, vol. 272, 23843-23850
- **JOHNSON ; TOFT.** Binding of p23 and hsp90 during assembly with the progesterone receptor. *Mol. Endocrinol.,* 1995, vol. 9, 670-678
- **HARTL ; HAYER-HARTL.** Molecular chaperones in the cytosol: from nascent chain to folded protein. *Science,* 2002, vol. 195, 1852-1858

- **HEGDE et al.** Short circuiting stress protein expression via a tyrosine kinase inhibitor, herbimycin A. *J. Cell Physiol.,* 1995, vol. 165, 186-200
- **HUSTERT et al.** The genetic determinants of the CYP3A5 polymorphism. *Pharmacogenetics,* 2001, vol. 11, 773-779
- **KELLAND et al.** DT-Diaphorase expression and tumor cell sensitivity to 17-allylamino, 17-demethoxygeldanamycin, an inhibitor of heat shock protein 90. *J. Natl. Cancer Inst.,* 1999, vol. 91, 1940-1949
- **KUEHL et al.** Sequence diversity in CYP3A promoters and characterization of the genetic basis of polymorphic CYP3A5 expression. *Nat. Genet.,* 2001, vol. 27, 383-391
- **LAWSON et al.** Geldanamycin, an hsp90/GRP94-binding drug, induces increased transcription of endoplasmic reticulum (ER) chaperones via the ER stress pathway. *J. Cell Physiol.,* 1998, vol. 174, 170-178
- **LIN et al.** Co-regulation of CYP3A4 and CYP3A5 and contribution to hepatic and intestinal midazolam metabolism. *Mol. Pharmacol.,* 2002, vol. 62, 162-172
- **MORIMOTO et al.** The heat-shock response: regulation and function of heat-shock proteins and molecular chaperones. *Essays Biochem,* 1997, vol. 32, 17-29
- **MUNSTER et al.** Phase I trial of 17-(allylamino)-17-demethoxygeldanamycin (17-AAG) in patients with advanced solid malignancies. *Proc. Am. Soc. Clin. Oncol,* 2001, vol. 83a
- **MUNSTER et al.** Modulation of Hsp90 function by ansamycins sensitizes breast cancer cells to chemotherapy-induced apoptosis in an RB- and schedule-dependent manner. *Clin. Cancer Res.,* 2001, vol. 7, 2228-2236
- **MURAKAMI et al.** Induction of hsp 72/73 by herbimycin A, an inhibitor of transformation by tyrosine kinase oncogenes. *Exp. Cell Res.,* 1991, vol. 195, 338-344
- **PRATT ; TOFT.** Steroid receptor interactions with heat shock protein and immunophilin chaperones. *Endocr. Rev.,* 1997, vol. 18, 306-60
- **PRODROMOU et al.** Identification and structural characterization of the ATP/ADP-binding site in the Hsp90 molecular chaperone. *Cell,* 1997, vol. 90, 65-75
- **RICHTER ; BUCHNER.** Hsp90: chaperoning signal transduction. *J. Cell. Physiol.,* 2001, vol. 188, 281-290
- **ROSVOLD et al.** Identification of an NAD(P)H:quinone oxidoreductase polymorphism and its association with lung cancer and smoking. *Pharmacogenetics,* 1995, vol. 5, 199-206
- **SCHNEIDER et al.** Pharmacologic shifting of a balance between protein refolding and degradation mediated by Hsp90. *Proc. Natl. Acad. Sci. USA,* 1996, vol. 93, 14536-14541
- **SCHNUR et al.** erbB-2 oncogene inhibition by geldanamycin derivatives: synthesis, mechanism of action, and structure-activity relationships. *J. Med. Chem.,* 1995, vol. 38, 3813-20
- **SCHNUR et al.** Inhibition of the oncogene product p185erbB-2 in vitro and in vivo by geldanamycin and dihydrogeldanamycin derivatives. *J. Med. Chem.,* 1995, vol. 38, 3806-3812
- **SMITH et al.** Progesterone receptor structure and function altered by geldanamycin, an hsp90-binding agent. *Mol. Cell BioL,* 1995, vol. 15, 6804-6812
- **SMITH et al.** Identification of a 60-kilodalton stress-related protein, p60, which interacts with hsp90 and hsp70. *Mol. Cell Biol.,* 1993, vol. 13, 869-876
- **STEBBINS et al.** Crystal structure of an Hsp90-geldanamycin complex: targeting of a protein chaperone by an antitumor agent. *Cell,* 1997, vol. 89, 239-250
- **TRAVER et al.** NAD(P)H:quinone oxidoreductase gene expression in human colon carcinoma cells: characterization of a mutation which modulates DT-diaphorase activity and mitomycin sensitivity. *Cancer Res.,* 1992, vol. 52, 797-802
- **WHITESELL et al.** Inhibition of heat shock protein HSP90-pp60v-src heteroprotein complex formation by benzoquinone ansamycins: essential role for stress proteins in oncogenic transformation. *Proc. Natl Acad. Sci. USA,* 1994, vol. 91, 8324-8328
- **YOUNG et al.** Hsp90: a specialized but essential protein-folding tool. *J. Cell Biol.,* 2001, vol. 154, 267-273
- **ZOU et al.** Repression of heat shock transcription factor HSF1 activation by HSP90 (HSP90 complex) that forms a stress-sensitive complex with HSF1. *Cell,* 1998, vol. 94, 471-480
- **MÜNSTER et al.** Modulation of Hsp90 function by ansamycins sensitizes breast cancer cells to chemotherapy induced apoptosis in an RB- and schedule-dependent manner. *Clinical Cancer Research,* 2001, vol. 7, 2228-2236
- **CITRI et al.** Drug-induced ubiquitylation and degradation of ErbB receptor tyrosine kinases: implications for cancer chemotherapy. *EMBO Journal,* 2002, vol. 21, 2407-2417
- **MICHAEL L. ; GROSSBARD, ED.** Monoclonal Antibody-Based Therapy of Cancer. 1998